# EUROPEAN PATENT SPECIFICATION

(11) **EP 3 508 242 B1**
(45) Date of publication and mention of the grant of the patent: **11.01.2023**
(21) Application number: 16836041.0
(22) Date of filing: 01.09.2016
(51) Int. Cl.: A61M 25/00

(54) **CATHETER**
KATHETER
CATHÉTER

(43) Date of publication of application: 10.07.2019
(73) Proprietor: ASAHI INTECC CO., LTD., Seto-shi, Aichi 489-0071 (JP)
(72) Inventor: SHIMIZU Hirotomo, Seto-shi, Aichi 489-0071 (JP)
(74) Representative: Winter, Brandl - Partnerschaft mbB
(86) International application number: PCT/JP2016/075683
(87) International publication number: WO 2018/042596

(56) References cited:
- EP-A1- 2 174 685
- EP-A1- 2 213 325
- EP-A2- 2 923 724
- WO-A1-2009/085486
- JP-A- H 078 563
- JP-A- 2008 229 160
- JP-A- 2010 137 095
- US-A- 5 702 373
- US-A- 5 769 830

## Description

### Field

The present invention relates to a catheter in which a tip is joined to a distal end of a catheter shaft. Background

When a stenosis site or a blocked portion is formed in a blood vessel, a bile duct, a pancreatic duct, and the like, a flow of blood, gall (bile), pancreatic juice, and the like is disrupted. As a method of treating such a stenosis site or a blocked portion, a treatment using a catheter is performed widely.

Generally in a catheter, a flexible tip is joined to a distal end of a catheter shaft including a reinforcing layer (see the following Patent Literature 1, for example). In this manner, the catheter shaft having rigidity maintains operability of the catheter, while the flexible tip reduces damages to duct walls of a blood vessel, a bile duct, a pancreatic duct, and the like when the catheter is inserted.

However, in such a catheter, the tip is only joined to the distal end of the catheter shaft. The joining strength between the catheter shaft and the tip is small, which has caused a problem that the tip is removed from the catheter shaft when a technician operates the catheter in the state where the tip is caught by a stenosis site or a stenosis site.

Moreover, when the catheter is inserted into a strongly bent blood vessel, bile duct, pancreatic duct, or the like, stress is concentrated on a boundary portion between the tip and the catheter shaft, which has also caused a problem that the tip is broken at the boundary portion.
As further, Patent Literature 2 relates to a catheter assembly having at least a more distal section made up preferably of an inner liner and an outer covering and having a superelastic alloy braid located between the liner and interior to the outer covering and a more proximal section comprising a stiff polymeric or metallic tubing member, possibly with an inner lubricious liner.
Patent Literature 3 discloses a catheter body including a hollow coil whose outer and inner surfaces are coated with outer and inner resin layers. A resin tip is provided at a distal end of the catheter body. The tip has a tapered section at a distal end of the tip. Also, an axially extending braid is embedded in the catheter body and the tip to extend from the catheter body to the tip.
Patent Literature 4 relates to a catheter including a catheter shaft extending from a proximal end to a distal end and comprising a main body and a distal end portion, and a distal end tip. The main body includes an inner layer defining a first space, a coil body wound around an outer periphery of the inner layer and made of a metal, and an outer layer that covers an outer periphery of the coil body. The distal end tip defines a second space that communicates with the first space. The distal end tip is provided at a distal end of the catheter shaft, and is made of a metal. The distal end portion includes the coil body and the outer layer. The distal end tip is bonded to a distal end of the coil body and an inner surface of a distal end portion of the coil body.
Patent Literature 5 relates to a catheter shaft with multiple reinforcing layers and method of its manufacture.

### Citation List

### Patent Literature

Patent Literature 1: US Patent No. 5769830
Patent Literature 2: US 5702373 A
Patent Literature 3: EP 2174685 A1
Patent Literature 4: EP 2923724 A2
Patent Literature 5: WO 2009/085486 A1

### Summary

### Technical Problem

In view of such aspects, the present invention aims at providing a catheter in which a tip is hardly removed from a catheter shaft or a break hardly occurs at a boundary portion between the tip and the catheter shaft. Solution to Problem

The above-described problem is solved by the following means.

The first aspect of the invention is a catheter that includes a catheter shaft having an inner layer, a first reinforcing layer wound on an outer periphery of the inner layer, an intermediate layer covering the first reinforcing layer, a second reinforcing layer wound on an outer periphery of the intermediate layer, and an outer layer covering the second reinforcing layer, and a tip joined to a distal end of the catheter shaft, in which the tip includes a rear end portion extended in an axis direction and joined to the intermediate layer and the outer layer, between the first reinforcing layer and the second reinforcing layer.

The second aspect of the invention is the catheter, in which the intermediate layer includes an uneven outer peripheral surface, the outer layer includes an uneven inner peripheral surface, and the rear end portion of the tip is joined to at least one of the outer peripheral surface of the intermediate layer and the inner peripheral surface of the outer layer.

The third aspect of the invention is the catheter, in which the thickness of the rear end portion of the tip is increased toward a distal end direction.

### Advantageous Effects of Invention

In the catheter according to the first aspect of the invention, the tip includes the rear end portion extended in the axis direction and joined to the intermediate layer and the outer layer, between the first reinforcing layer and the second reinforcing layer. This increases joining strength between the catheter shaft and the tip (in other words, joining strength between the intermediate layer and the tip or/and joining strength between the outer layer and the tip). As a result, it is possible to prevent the tip from being removed easily from the catheter shaft. Moreover, the rear end portion extended in the axis direction can reduce a risk that the tip is broken at the boundary portion even when stress is concentrated on the boundary portion between the tip and the catheter shaft.

In the catheter according to the second aspect of the invention, the rear end portion of the tip is joined to at least one of the uneven outer peripheral surface of the intermediate layer and the uneven inner peripheral surface of the outer layer. Thus, the anchoring effect increases joining strength between the catheter shaft and the tip (in other words, joining strength between the intermediate layer and the tip or/and joining strength between the outer layer and the tip), and it is possible to further prevent the tip from being removed easily from the catheter shaft.

In the catheter according to the third aspect of the invention, the thickness of the rear end portion of the tip is increased toward the distal end direction. Thus, it is possible to further reduce a risk that the tip is broken at the boundary portion even when stress is concentrated on the boundary portion between the tip and the catheter shaft.

### Brief Description of Drawings

FIG. 1 is a diagram illustrating an entire view of a catheter according to a first embodiment.
FIG. 2 is an enlarged section view of an A part of FIG. 1.
FIG. 3 is a section view illustrating a part of a catheter according to a second embodiment.
FIG. 4 is a section view illustrating a part of a catheter according to a third embodiment.
FIG. 5 is a section view illustrating a part of a catheter according to a fourth embodiment, and is a first modification of FIG. 2.
FIG. 6 is a section view illustrating a part of a catheter according to a fifth embodiment, and is a first modification of FIG. 3.
FIG. 7 is a section view illustrating a part of a catheter according to a sixth embodiment, and is a first modification of FIG. 4.
FIG. 8 is a section view illustrating a part of a catheter according to a seventh embodiment, and is a second modification of FIG. 2.
FIG. 9 is a section view illustrating a part of a catheter according to an eighth embodiment, and is a second modification of FIG. 3.
FIG. 10 is a section view illustrating a part of a catheter according to a ninth embodiment, and is a second modification of FIG. 4.
FIG. 11 is a section view illustrating a part of a catheter according to a tenth embodiment, and is a modification of FIG. 5.
FIG. 12 is a section view illustrating a part of a catheter according to an eleventh embodiment, and is a modification of FIG. 6.
FIG. 13 is a section view illustrating a part of a catheter according to a twelfth embodiment, and is a modification of FIG. 7.
FIG. 14 is a modification of FIG. 8.
FIG. 15 is a modification of FIG. 11.

### Description of Embodiments

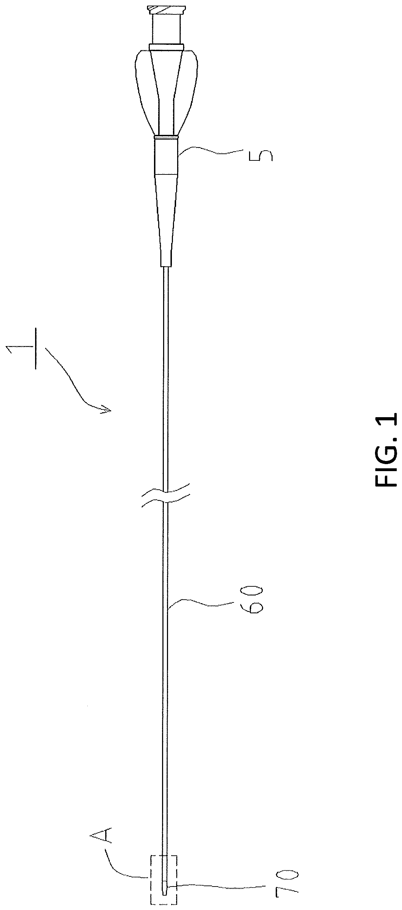

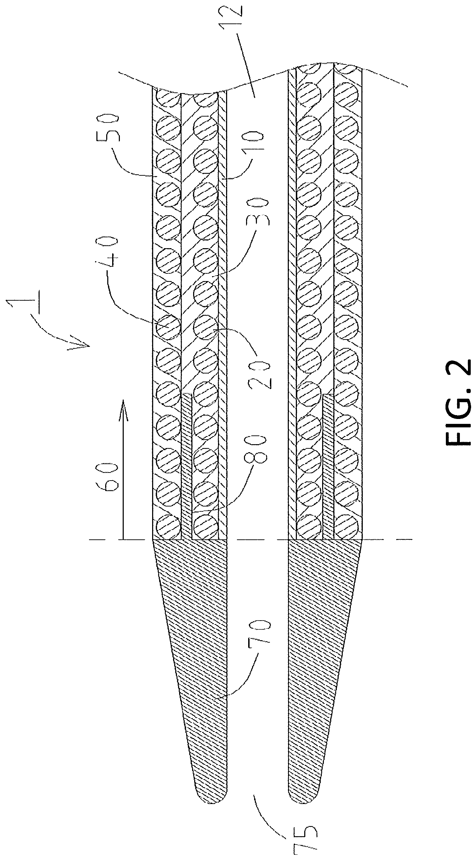

A catheter 1 according to a first embodiment will be described with reference to FIG. 1 to FIG. 2. In FIG. 1 and FIG. 2, the left side of the drawings is a distal end side(far side) to be inserted in a body, while the right side is a rear end side (near side) to be operated by a technician such as a physician. FIG. 2 is an enlarged section view of an A part of FIG. 1.

The catheter 1 is a catheter used for treating a stenosis site or a blocked portion. As illustrated in FIG. 1, the catheter 1 mainly includes a catheter shaft 60, a tip 70 joined to a distal end of the catheter shaft 60, and a connector 5 joined to a rear end of the catheter shaft 60.

The catheter shaft 60 includes, in the order from the inner side in a radial direction, an inner layer 10, a first coil body 20 that is a first reinforcing layer wound on an outer periphery of the inner layer 10, an intermediate layer 30 covering the first coil body 20, a second coil body 40 that is a second reinforcing layer wound on an outer periphery of the intermediate layer 30, and an outer layer 50 covering the second coil body 40, as illustrated in FIG. 2.

The inner layer 10 is formed of resin, and forms a lumen 12 into which a guide wire or another catheter is inserted. The resin material forming the inner layer 10 is not particularly limited. In the first embodiment, polytetrafluoroethylene (PTFE) is used.

The first coil body 20 that is the first reinforcing layer is formed on the outer periphery of the inner layer 10. Such a first coil body 20 is wound in a clockwise direction toward the distal end side. As the material forming the first coil body 20, stainless steel (SUS304) is used in the first embodiment. However, the embodiment is not limited thereto. For example, there may be used not only a metal material such as tungsten or an Ni-Ti alloy but also a resin material such as reinforced plastic (PEEK).

The intermediate layer 30 formed of resin is formed on the outer periphery of the first coil body 20, and covers the inner layer 10 and the first coil body 20. The resin material forming the intermediate layer 30 is not particularly limited, and polyamide, polyamide elastomer, polyester, polyurethane, and the like can be used.

The second coil body 40 that is the second reinforcing layer is formed on the outer periphery of the intermediate layer 30. This second coil body 40 is wound in a counterclockwise direction toward the distal end side, which is an opposite direction to the winding direction of the first coil body 20. As the material forming the second coil body 40, there may be used not only a metal material such as stainless steel (SUS304), tungsten or an Ni-Ti alloy but also a resin material such as reinforced plastic (PEEK), for example, similarly to the first coil body 20.

The outer layer 50 formed of resin is formed on the outer periphery of the second coil body 40, and covers the intermediate layer 30 and the second coil body 40. The resin material forming the outer layer 50 is not particularly limited, and polyamide, polyamide elastomer, polyester, polyurethane, and the like can be used, similarly to the intermediate layer 30.

A tip 70 formed of resin is joined to the distal end of the catheter shaft 60. The tip 70 is a cylindrical member having a distal end opening 75 communicating with the lumen 12. The resin forming the tip 70 is not particularly limited, and polyurethane, polyurethane elastomer, and the like are used. Moreover, the tip 70 may contain radiopaque powder. For example, when the tip 70 contains radiopaque powder (e.g., tungsten powder) in a range of about 65 w% to about 90 w%, the technician such as a physician can accurately grasp a position of the catheter 1 in coronary angiography.

This tip 70 includes a rear end portion 80 extended in an axis direction and joined to the intermediate layer 30 and the outer layer 50, between the first coil body 20 that is the first reinforcing layer and the second coil body 40 that is the second reinforcing layer (see FIG. 2). In this manner, the rear end portion 80 extended in the axis direction is joined to the intermediate layer 30 and the outer layer 50, which increases joining strength between the intermediate layer 30 and the tip 70 and joining strength between the outer layer 50 and the tip 70. In other words, the joining strength between the catheter shaft 60 and the tip 70 is increased. As a result, it is possible to prevent the tip 70 from being removed easily from the catheter shaft 60.

Moreover, even when stress is concentrated on the boundary portion between the tip 70 and the catheter shaft 60, the rear end portion 80 extended in the axis direction can reduce a risk that the tip 70 is broken at the boundary portion. Furthermore, when the technician rotates the catheter 1 in a clockwise direction, the first coil body 20 wound in a clockwise direction is loosened to expand in a radial direction, while the second coil body 40 wound in a counterclockwise direction is tightened to shrink in a radial direction. Thus, even when the catheter 1 is operated in the state where the tip 70 is caught by a stenosis site or a blocked portion, the rear end portion 80 of the tip 70 is pressed by the first coil body 20 and the second coil body 40 by operating the catheter 1 while rotating it in a clockwise direction, which makes it possible that the tip 70 is hardly removed from the catheter shaft 60.

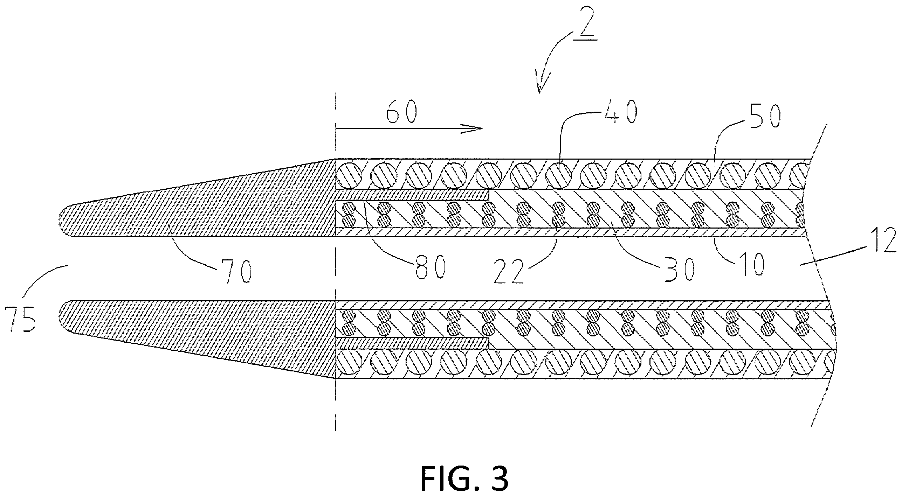

Next, a catheter 2 of the second embodiment will be described with reference to FIG. 3. Explaining only a difference from the catheter 1 illustrated in FIG. 2, the catheter 2 includes a first braid 22 that is a first reinforcing layer, instead of the first coil body 20.

In this first braid 22, first wire and second wire are mutually woven in a net form (mesh form). In the second embodiment, the total of 16 pieces (8 pieces x 8 pieces) of wire including eight pieces of first wire and eight pieces of second wire are woven alternately.

The material of the first wire and the second wire forming the first braid 22 may be same or different. In the second embodiment, the first wire formed of tungsten and the second wire formed of stainless steel (SUS304) are used. However, the embodiment is not particularly limited thereto, and a resin material other than metal (e.g., reinforced plastic) may be used.

The catheter 2 includes the rear end portion 80 extended in the axis direction and joined to the intermediate layer 30 and the outer layer 50, between the first braid 22 that is the first reinforcing layer and the second coil body 40 that is the second reinforcing layer (see FIG. 3). In this manner, the rear end portion 80 extended in the axis direction is joined to the intermediate layer 30 and the outer layer 50, which increases joining strength between the intermediate layer 30 and the tip 70 and joining strength between the outer layer 50 and the tip 70, similarly to the catheter 1. In other words, the joining strength between the catheter shaft 60 and the tip 70 is increased. As a result, it is possible to prevent the tip 70 from being removed easily from the catheter shaft 60.

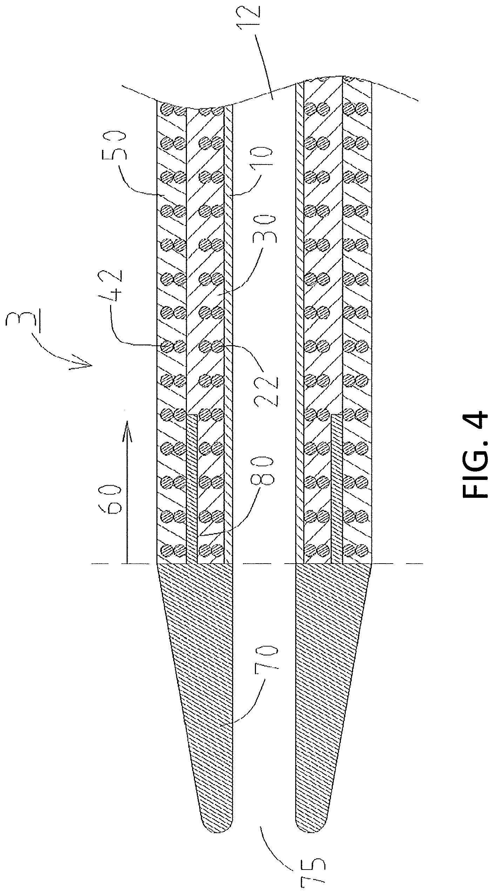

Next, a catheter 3 of the third embodiment will be described with reference to FIG. 4. Explaining only a difference from the catheter 2 illustrated in FIG. 3, the catheter 3 includes a second braid 42 that is the second reinforcing layer, instead of the second coil body 40.

In this second braid 42, first wire and second wire are mutually woven in a net form (mesh form), similarly to the first braid 22. In the third embodiment, the total of 16 pieces (8 pieces x 8 pieces) of wire including eight pieces of first wire and eight pieces of second wire are woven alternately.

The material of the first wire and the second wire forming the second braid 42 may be same or different. In the third embodiment, the first wire formed of tungsten and the second wire formed of stainless steel (SUS304) are used. However, the embodiment is not particularly limited thereto, and a resin material other than metal (e.g., reinforced plastic) may be used.

The catheter 3 includes the rear end portion 80 extended in the axis direction and joined to the intermediate layer 30 and the outer layer 50, between the first braid 22 that is the first reinforcing layer and the second braid 42 that is the second reinforcing layer (see FIG. 4). In this manner, the rear end portion 80 extended in the axis direction is joined to the intermediate layer 30 and the outer layer 50, which increases joining strength between the intermediate layer 30 and the tip 70 and joining strength between the outer layer 50 and the tip 70, similarly to the catheters 1, 2. In other words, the joining strength between the catheter shaft 60 and the tip 70 is increased. As a result, it is possible to prevent the tip 70 from being removed easily from the catheter shaft 60.

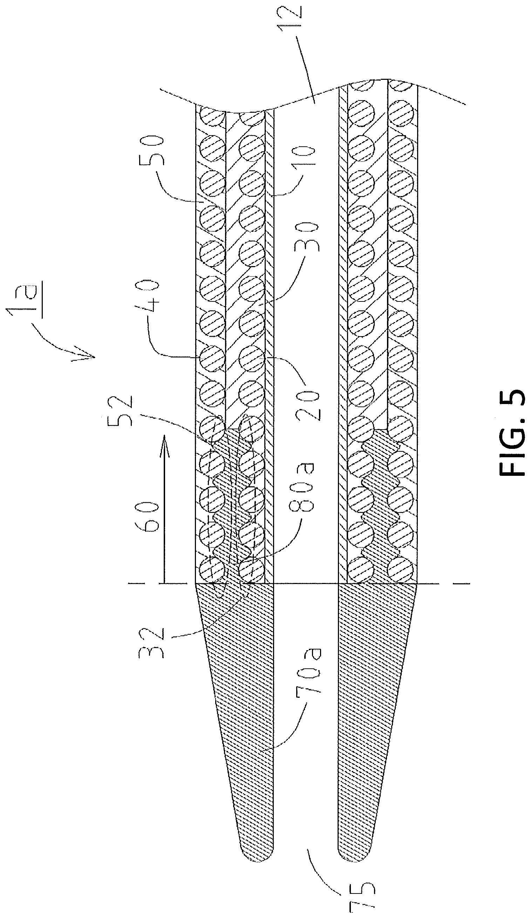

Next, a catheter 1a of the fourth embodiment will be described with reference to FIG. 5. Explaining only a difference from the catheter 1 illustrated in FIG. 2, in the catheter 1a, the intermediate layer 30 includes an uneven outer peripheral surface 32, and the outer layer 50 includes an uneven inner peripheral surface 52 (see FIG. 5). A tip 70a includes a rear end portion 80a joined to the outer peripheral surface 32 of the intermediate layer 30 and the inner peripheral surface 52 of the outer layer 50, between the first coil body 20 that is the first reinforcing layer and the second coil body 40 that is the second reinforcing layer. The anchoring effect between the rear end portion 80a and the outer peripheral surface 32 of the intermediate layer 30 and the anchoring effect between the rear end portion 80a and the inner peripheral surface 52 of the outer layer 50 increase joining strength between the intermediate layer 30 and the tip 70a and joining strength between the outer layer 50 and the tip 70a. In other words, the joining strength between the catheter shaft 60 and the tip 70a is increased. As a result, it is possible to further prevent the tip 70a from being removed easily from the catheter shaft 60.

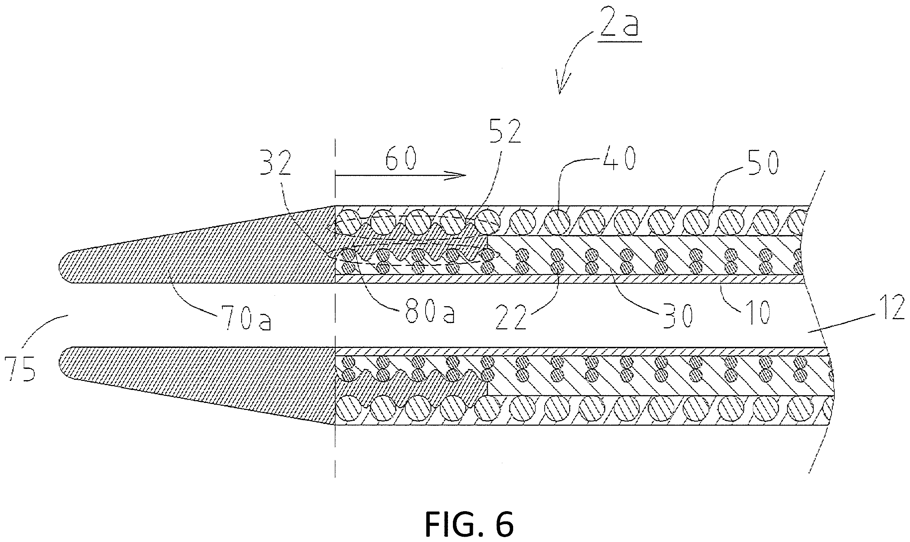

Next, a catheter 2a of the fifth embodiment will be described with reference to FIG. 6. Explaining only a difference from the catheter 2 illustrated in FIG. 3, in the catheter 2a, the intermediate layer 30 includes the uneven outer peripheral surface 32, and the outer layer 50 includes the uneven inner peripheral surface 52 (see FIG. 6). The tip 70a includes the rear end portion 80a joined to the outer peripheral surface 32 of the intermediate layer 30 and the inner peripheral surface 52 of the outer layer 50, between the first braid 22 that is the first reinforcing layer and the second coil body 40 that is the second reinforcing layer. The anchoring effect between the rear end portion 80a and the outer peripheral surface 32 of the intermediate layer 30 and the anchoring effect between the rear end portion 80a and the inner peripheral surface 52 of the outer layer 50 increase joining strength between the intermediate layer 30 and the tip 70a and joining strength between the outer layer 50 and the tip 70a. In other words, the joining strength between the catheter shaft 60 and the tip 70a is increased. As a result, it is possible to further prevent the tip 70a from being removed easily from the catheter shaft 60.

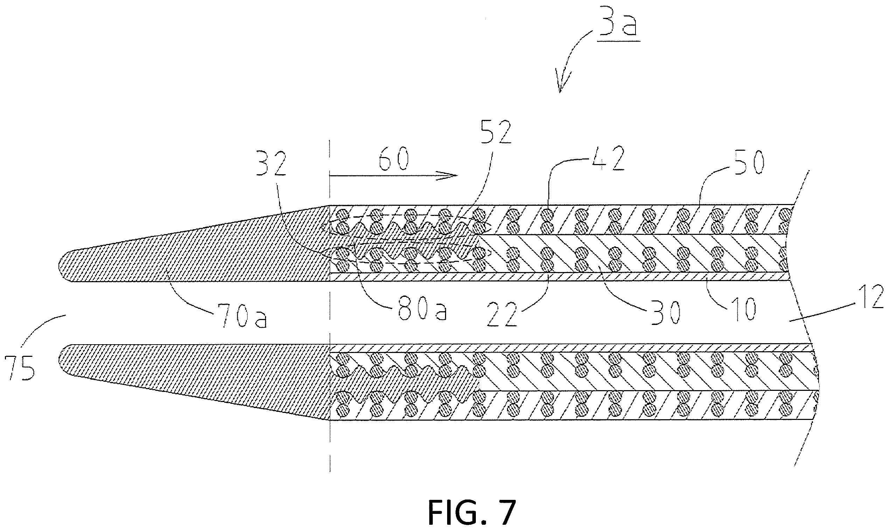

Next, a catheter 3a of the sixth embodiment will be described with reference to FIG. 7. Explaining only a difference from the catheter 3 illustrated in FIG. 4, in the catheter 3a, the intermediate layer 30 includes the uneven outer peripheral surface 32, and the outer layer 50 includes the uneven inner peripheral surface 52 (see FIG. 7). The tip 70a includes the rear end portion 80a joined to the outer peripheral surface 32 of the intermediate layer 30 and the inner peripheral surface 52 of the outer layer 50, between the first braid 22 that is the first reinforcing layer and the second braid 42 that is the second reinforcing layer. The anchoring effect between the rear end portion 80a and the outer peripheral surface 32 of the intermediate layer 30 and the anchoring effect between the rear end portion 80a and the inner peripheral surface 52 of the outer layer 50 increase joining strength between the intermediate layer 30 and the tip 70a and joining strength between the outer layer 50 and the tip 70a. In other words, the joining strength between the catheter shaft 60 and the tip 70a is increased. As a result, it is possible to further prevent the tip 70a from being removed easily from the catheter shaft 60.

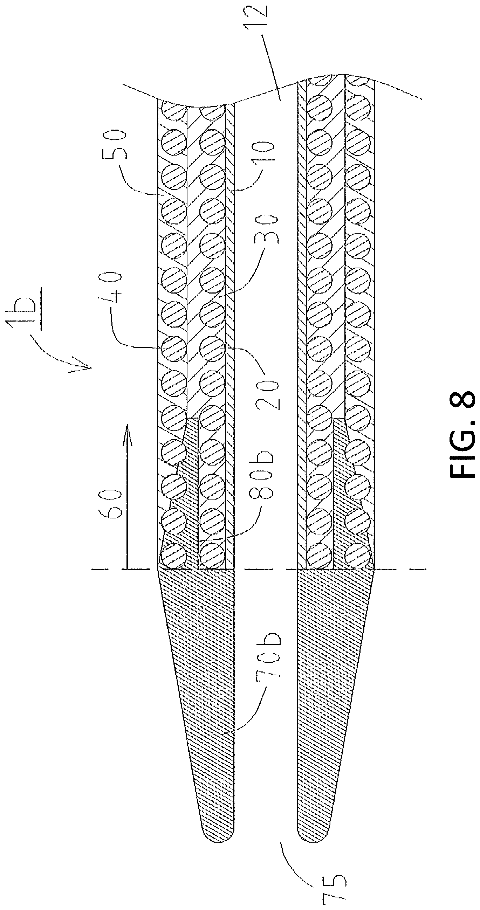

Next, a catheter 1b of the seventh embodiment will be described with reference to FIG. 8. Explaining only a difference from the catheter 1 illustrated in FIG. 2, in the catheter 1b, the thickness of a rear end portion 80b of a tip 70b is increased toward the distal end direction (in other words, the thickness of the rear end portion 80b is thinned toward the rear end direction). In this manner, the thickness of the rear end portion 80b is large at the boundary portion between the tip 70b and the catheter shaft 60, which can further reduce a risk that the tip 70b is broken at the boundary portion even when stress is concentrated on the boundary portion.

Moreover, the rear end portion 80b of the tip 70b covers a distal end portion of the second coil body 40 that is the second reinforcing layer (see FIG. 8). Thus, even when the technician operates the catheter 1b in the state where the tip 70b is caught by a stenosis site or a blocked portion, the anchoring effect between the rear end portion 80b of the tip 70b and the second coil body 40 can further reduce a risk that the tip 70b is removed from the catheter shaft 60.

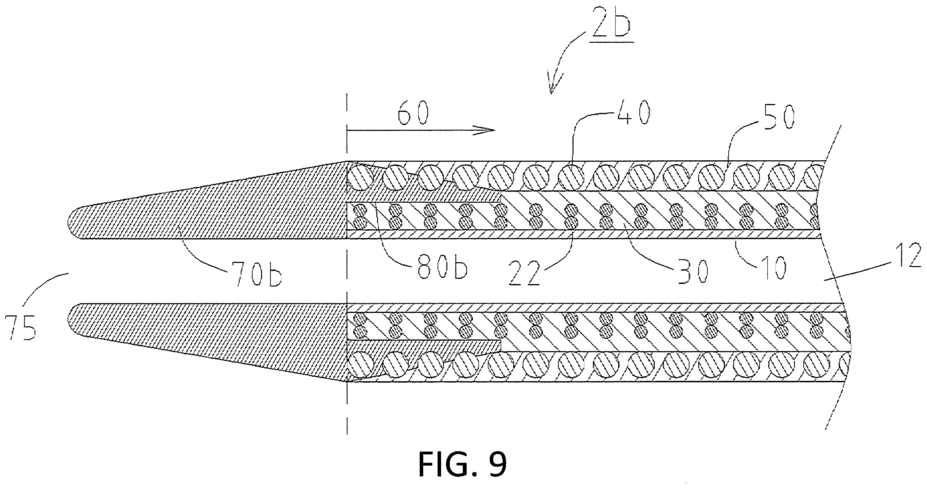

Next, a catheter 2b of the eighth embodiment will be described with reference to FIG. 9. Explaining only a difference from the catheter 2 illustrated in FIG. 3, in the catheter 2b, the thickness of the rear end portion 80b of the tip 70b is increased toward the distal end direction (in other words, the thickness of the rear end portion 80b is thinned toward the rear end direction). In this manner, the thickness of the rear end portion 80b is large at the boundary portion between the tip 70b and the catheter shaft 60, which can further reduce a risk that the tip 70b is broken at the boundary portion even when stress is concentrated on the boundary portion.

Moreover, the rear end portion 80b of the tip 70b covers a distal end portion of the second coil body 40 that is the second reinforcing layer (see FIG. 9). Thus, even when the technician operates the catheter 2b in the state where the tip 70b is caught by a stenosis site or a blocked portion, the anchoring effect between the rear end portion 80b of the tip 70b and the second coil body 40 can further reduce a risk that the tip 70b is removed from the catheter shaft 60.

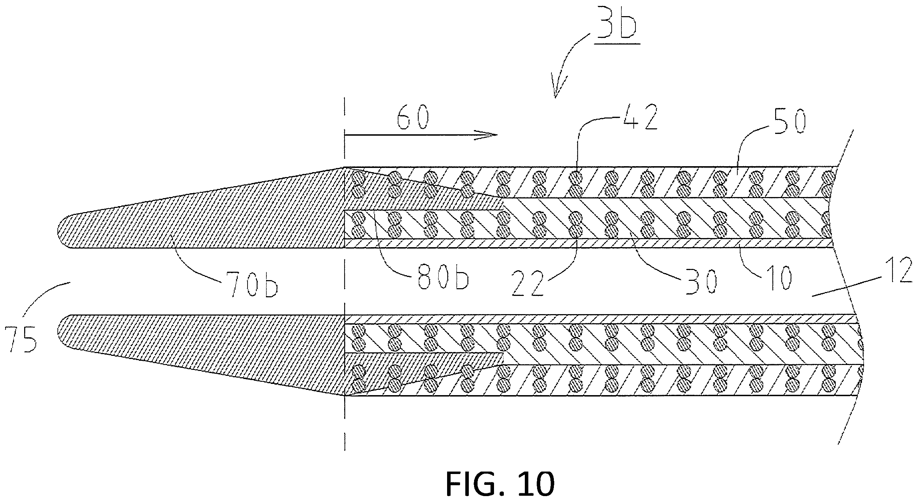

Next, a catheter 3b of the ninth embodiment will be described with reference to FIG. 10. Explaining only a difference from the catheter 3 illustrated in FIG. 4, in the catheter 3b, the thickness of the rear end portion 80b of the tip 70b is increased toward the distal end direction (in other words, the thickness of the rear end portion 80b is thinned toward the rear end direction). In this manner, the thickness of the rear end portion 80b is large at the boundary portion between the tip 70b and the catheter shaft 60, which can further reduce a risk that the tip 70b is broken at the boundary portion even when stress is concentrated on the boundary portion.

Moreover, the rear end portion 80b of the tip 70b covers a distal end portion of the second braid 42 that is the second reinforcing layer (see FIG. 10). Thus, even when the technician operates the catheter 3b in the state where the tip 70b is caught by a stenosis site or a blocked portion, the anchoring effect between the rear end portion 80b of the tip 70b and the second braid 42 can further reduce a risk that the tip 70b is removed from the catheter shaft 60.

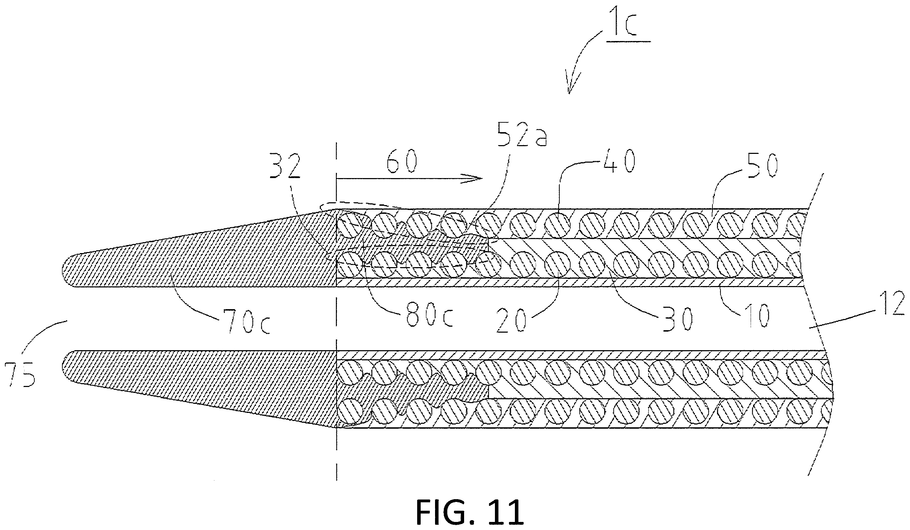

Next, a catheter 1c of the tenth embodiment will be described with reference to FIG. 11. Explaining only a difference from the catheter 1a illustrated in FIG. 5, in the catheter 1c, the outer layer 50 has a thickness thinned toward the distal end, and includes an inclined uneven inner peripheral surface 52a (see FIG. 11). Then, a tip 70c includes a rear end portion 80c joined to the outer peripheral surface 32 of the intermediate layer 30 and the inner peripheral surface 52a of the outer layer 50, between the first coil body 20 that is the first reinforcing layer and the second coil body 40 that is the second reinforcing layer. The thickness of the rear end portion 80c of the tip 70c is increased toward the distal end direction (in other words, the thickness of the rear end portion 80c is thinned toward the rear end direction).

In this manner, in the catheter 1c, the thickness of the rear end portion 80c is large at the boundary portion between the tip 70c and the catheter shaft 60, which can further reduce a risk that the tip 70c is broken at the boundary portion even when stress is concentrated on the boundary portion. Moreover, the anchoring effect between the rear end portion 80c and the outer peripheral surface 32 of the intermediate layer 30 and the anchoring effect between the rear end portion 80c and the inner peripheral surface 52a of the outer layer 50 further increase joining strength between the intermediate layer 30 and the tip 70c and joining strength between the outer layer 50 and the tip 70c. In other words, the joining strength between the catheter shaft 60 and the tip 70c is increased. As a result, it is possible to further prevent the tip 70c from being removed easily from the catheter shaft 60.

Moreover, the rear end portion 80c of the tip 70c covers a distal end portion of the second coil body 40 that is the second reinforcing layer (see FIG. 11). Thus, even when the technician operates the catheter 1c in the state where the tip 70c is caught by a stenosis site or a blocked portion, the anchoring effect between the rear end portion 80c of the tip 70c and the second coil body 40 can further reduce a risk that the tip 70c is removed from the catheter shaft 60.

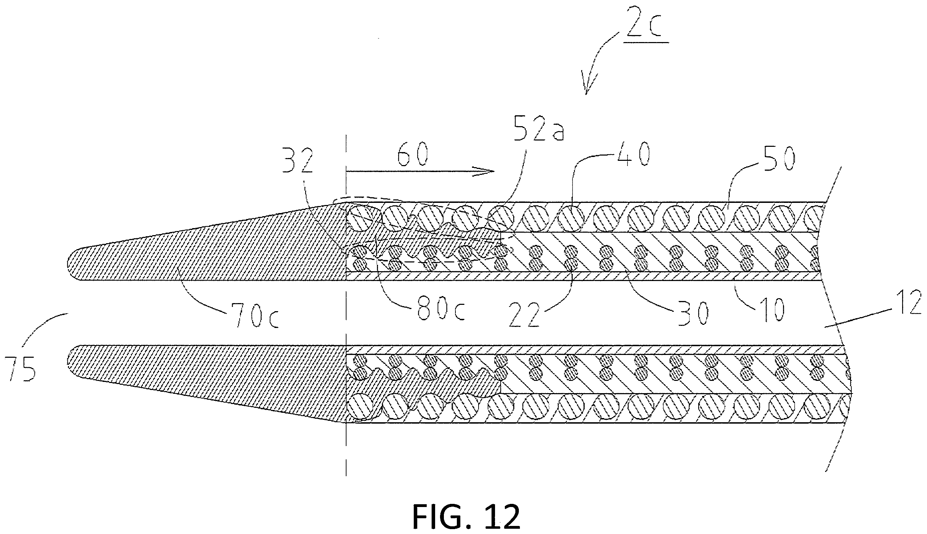

Next, a catheter 2c of the eleventh embodiment will be described with reference to FIG. 12. Explaining only a difference from the catheter 2a illustrated in FIG. 6, in the catheter 2c, the outer layer 50 has a thickness thinned toward the distal end, and includes the inclined uneven inner peripheral surface 52a (see FIG. 12). Then, the tip 70c includes the rear end portion 80c joined to the outer peripheral surface 32 of the intermediate layer 30 and the inner peripheral surface 52a of the outer layer 50, between the first braid 22 that is the first reinforcing layer and the second coil body 40 that is the second reinforcing layer. The thickness of the rear end portion 80c of the tip 70c is increased toward the distal end direction (in other words, the thickness of the rear end portion 80c is thinned toward the rear end direction).

In this manner, in the catheter 2c, the thickness of the rear end portion 80c is large at the boundary portion between the tip 70c and the catheter shaft 60, which can further reduce a risk that the tip 70c is broken at the boundary portion even when stress is concentrated on the boundary portion. Moreover, the anchoring effect between the rear end portion 80c and the outer peripheral surface 32 of the intermediate layer 30 and the anchoring effect between the rear end portion 80c and the inner peripheral surface 52a of the outer layer 50 further increase joining strength between the intermediate layer 30 and the tip 70c and joining strength between the outer layer 50 and the tip 70c. In other words, the joining strength between the catheter shaft 60 and the tip 70c is increased. As a result, it is possible to further prevent the tip 70c from being removed easily from the catheter shaft 60.

Moreover, the rear end portion 80c of the tip 70c covers a distal end portion of the second coil body 40 that is the second reinforcing layer (see FIG. 12). Thus, even when the technician operates the catheter 2c in the state where the tip 70c is caught by a stenosis site or a blocked portion, the anchoring effect between the rear end portion 80c of the tip 70c and the second coil body 40 can further reduce a risk that the tip 70c is removed from the catheter shaft 60.

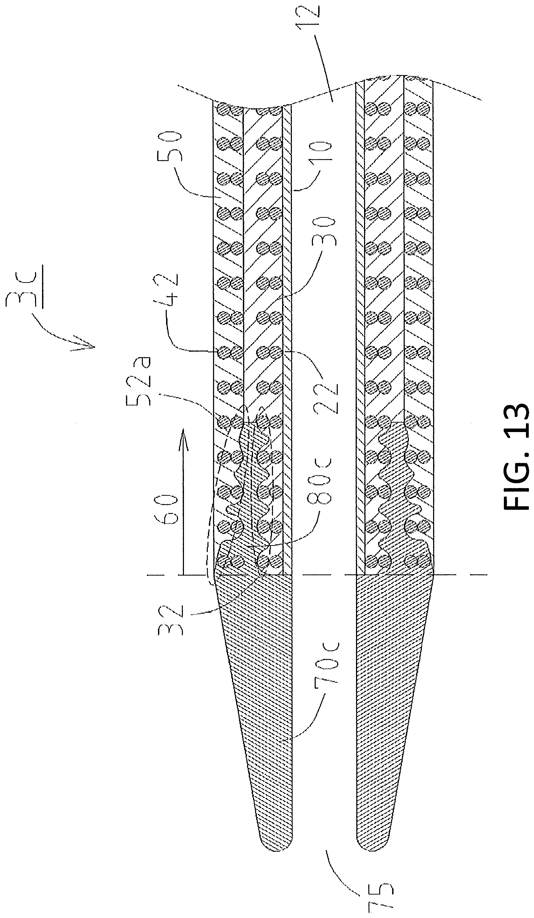

Next, a catheter 3c of the twelfth embodiment will be described with reference to FIG. 13. Explaining only a difference from the catheter 3a illustrated in FIG. 7, in the catheter 3c, the outer layer 50 has a thickness thinned toward the distal end, and includes the inclined uneven inner peripheral surface 52a (see FIG. 13). Then, the tip 70c includes the rear end portion 80c joined to the outer peripheral surface 32 of the intermediate layer 30 and the inner peripheral surface 52a of the outer layer 50, between the first braid 22 that is the first reinforcing layer and the second braid 42 that is the second reinforcing layer. The thickness of the rear end portion 80c of the tip 70c is increased toward the distal end direction (in other words, the thickness of the rear end portion 80c is thinned toward the rear end direction).

In this manner, in the catheter 3c, the thickness of the rear end portion 80c is large at the boundary portion between the tip 70c and the catheter shaft 60, which can further reduce a risk that the tip 70c is broken at the boundary portion even when stress is concentrated on the boundary portion. Moreover, the anchoring effect between the rear end portion 80c and the outer peripheral surface 32 of the intermediate layer 30 and the anchoring effect between the rear end portion 80c and the inner peripheral surface 52a of the outer layer 50 can further increase joining strength between the intermediate layer 30 and the tip 70c and joining strength between the outer layer 50 and the tip 70c. In other words, the joining strength between the catheter shaft 60 and the tip 70c is increased. As a result, it is possible to further prevent the tip 70c from being removed easily from the catheter shaft 60.

Moreover, the rear end portion 80c of the tip 70c covers a distal end portion of the second braid 42 that is the second reinforcing layer (see FIG. 13). Thus, even when the technician operates the catheter 3c in the state where the tip 70c is caught by a stenosis site or a blocked portion, the anchoring effect between the rear end portion 80c of the tip 70c and the second braid 42 can further reduce a risk that the tip 70c is removed from the catheter shaft 60.

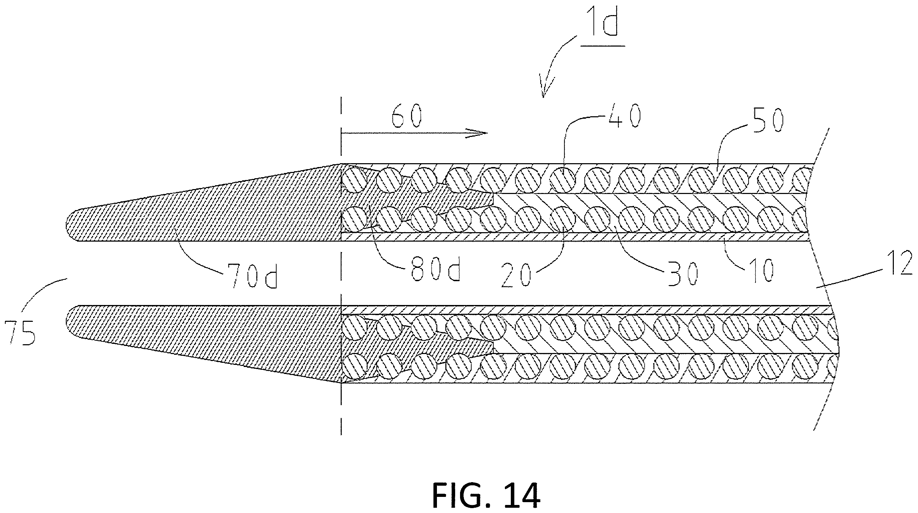

Note that in the catheter 1b illustrated in FIG. 8, the thickness of the rear end portion 80b of the tip 70b is increased toward the distal end direction only on the side of the second reinforcing layer (second coil body 40). However, the embodiment is not limited thereto. As illustrated in a catheter 1d of FIG. 14, the thickness of a rear end portion 80d of a tip 70d may be increased toward the distal end direction not only on the side of the second reinforcing layer (second coil body 40) but also on the side of the first reinforcing layer (first coil body 20). By contrast, the thickness of the rear end portion 80d of the tip 70d may be increased toward the distal end direction only on the side of the first reinforcing layer (first coil body 20).

Similarly, in the catheters 2b, 3b illustrated in FIG. 9 and FIG. 10, the thickness of the rear end portion 80b of the tip 70b may be increased toward the distal end direction not only on the side of the second reinforcing layer (second coil body 40 or second braid 42) but also on the side of the first reinforcing layer (first braid 22). By contrast, the thickness of the rear end portion 80b of the tip 70b may be increased toward the distal end direction only on the side of the first reinforcing layer (first braid 22).

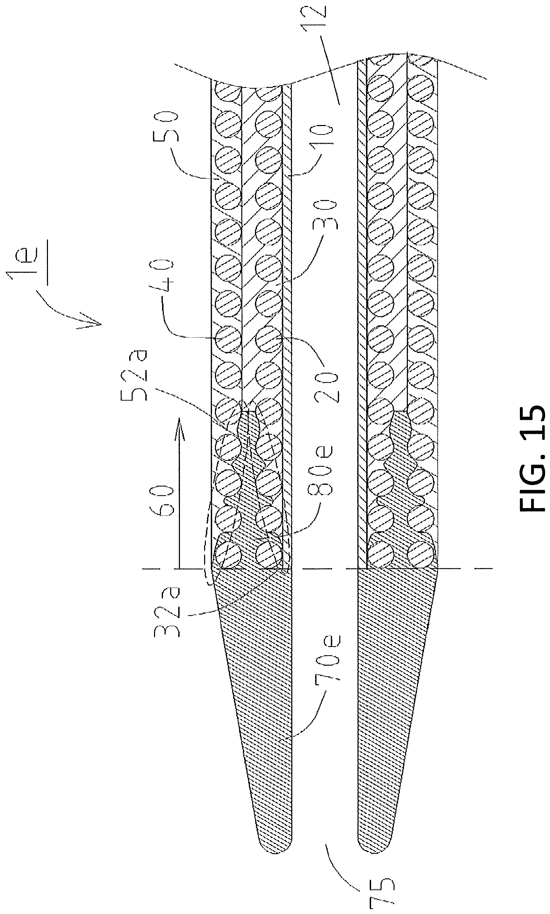

Note that in the catheter 1c illustrated in FIG. 11, the thickness of the rear end portion 80c of the tip 70c is increased toward the distal end direction only on the side of the second reinforcing layer (second coil body 40). However, the embodiment is not limited thereto. As illustrated in a catheter 1e of FIG. 15, the thickness of a rear end portion 80e of a tip 70e may be increased toward the distal end direction not only on the side of the second reinforcing layer (second coil body 40) but also on the side of the first reinforcing layer (first coil body 20). In the catheter 1e, the intermediate layer 30 includes an inclined uneven outer peripheral surface 32a, and the outer layer 50 includes the inclined uneven inner peripheral surface 52a (see FIG. 15). By contrast, the thickness of the rear end portion 80d of the tip 70d may be increased toward the distal end direction only on the side of the first reinforcing layer (first coil body 20).

Similarly, in the catheters 2c, 3c illustrated in FIG. 12 and FIG. 13, the thickness of the rear end portion 80c of the tip 70c may be increased toward the distal end direction not only on the side of the second reinforcing layer (second coil body 40 or second braid 42) but also on the side of the first reinforcing layer (first braid 22). By contrast, the thickness of the rear end portion 80c of the tip 70c may be increased toward the distal end direction only on the side of the first reinforcing layer (first braid 22) .

In the above description, there are exemplified the first coil body 20 or the first braid 22 as the first reinforcing layer, and the second coil body 40 or the second braid 42 as the second reinforcing layer. However, the embodiment is not limited thereto, and there may be used, as the first reinforcing layer or the second reinforcing layer, a metal hypo tube with a helical groove.

Moreover, in the above description, the rear end portions 80, 80a, 80b, 80c, 80d, 80e of the tips 70, 70a, 70b, 70c, 70d, 70e are joined to the intermediate layer 30 and the outer layer 50. Furthermore, the rear end portions 80a, 80c, 80e of the tips 70a, 70c, 70e are joined to the uneven outer peripheral surfaces 32, 32a of the intermediate layer 30 and the uneven inner peripheral surfaces 52, 52a of the outer layer 50. However, the embodiment is not limited thereto, and they may be joined to at least one of the uneven outer peripheral surfaces 32, 32a of the intermediate layer 30 and the uneven inner peripheral surfaces 52, 52a of the outer layer 50.

In addition, in the section views illustrated in FIG. 2 to FIG. 15, the rear end portions 80, 80a, 80b, 80c, 80d, 80e of the tips 70, 70a, 70b, 70c, 70d, 70e are formed on both the upper and lower sides (in other words, on the entire periphery) between the first reinforcing layer (first coil body 20 or first braid 22) and the second reinforcing layer (second coil body 40 or second braid 42). However, the embodiment is not limited thereto, and they may be formed on only one side (in other words, at a certain part).

### Reference Signs List

- 1 to 3c, 1d, 1e: catheter
- 5: connector
- 10: inner layer
- 12: lumen
- 20: first reinforcing layer (first coil body)
- 22: first reinforcing layer (first braid)
- 30: intermediate layer
- 40: second reinforcing layer (second coil body)
- 42: second reinforcing layer (second braid)
- 50: outer layer
- 60: catheter shaft
- 70, 70a, 70b, 70c, 70d, 70e: tip
- 75: distal end opening portion
- 80, 80a, 80b, 80c, 80d, 80e: rear end portion

## Claims

1. A catheter, comprising:
a catheter shaft (60) including
an inner layer (10);
a first reinforcing layer (20/22) wound on an outer periphery of the inner layer (10);
an intermediate layer (30) that covers the first reinforcing layer (20/22);
a second reinforcing layer (40/42) wound on an outer periphery of the intermediate layer (30); and
an outer layer (50) that covers the second reinforcing layer (40/42); and
a tip (70) joined to a distal end of the catheter shaft, **characterized in that**
the tip (70) includes a rear end portion (80) extended in an axis direction and joined to the intermediate layer (30) and the outer layer (50), between the first reinforcing layer (20/22) and the second reinforcing layer (40/42).

2. The catheter according to claim 1, wherein
the intermediate layer (30) includes an uneven outer peripheral surface,
the outer layer (50) includes an uneven inner peripheral surface, and
the rear end portion (80) of the tip (70) is joined to at least one of the outer peripheral surface of the intermediate layer (30) and the inner peripheral surface of the outer layer (50).

3. The catheter according to claim 1 or claim 2, wherein
a thickness of the rear end portion (80) of the tip (70) is increased toward a distal end direction.

## Patentansprüche

1. Katheter, umfassend:
einen Katheterschaft (60), umfassend
eine innere Schicht (10);
eine erste Verstärkungsschicht (20/22), die auf einen äußeren Umfang der inneren Schicht (10) gewickelt ist;
eine Zwischenschicht (30), die die erste Verstärkungsschicht (20/22) bedeckt;
eine zweite Verstärkungsschicht (40/42), die auf einen äußeren Umfang der Zwischenschicht (30) gewickelt ist; und
eine äußere Schicht (50), die die zweite Verstärkungsschicht (40/42) bedeckt; und
eine Spitze (70), die mit einem distalen Ende des Katheterschafts verbunden ist, **dadurch gekennzeichnet, dass**
die Spitze (70) einen hinteren Endabschnitt (80) umfasst, der sich in einer Achsenrichtung erstreckt und mit der Zwischenschicht (30) und der äußeren Schicht (50) zwischen der ersten Verstärkungsschicht (20/22) und der zweiten Verstärkungsschicht (40/42) verbunden ist.

2. Katheter nach Anspruch 1, wobei
die Zwischenschicht (30) eine unebene äußere Umfangsfläche umfasst,
die äußere Schicht (50) eine unebene innere Umfangsfläche umfasst, und
der hintere Endabschnitt (80) der Spitze (70) mit mindestens einem von der äußeren Umfangsfläche der Zwischenschicht (30) und der inneren Umfangsfläche der äußeren Schicht (50) verbunden ist.

3. Katheter nach Anspruch 1 oder Anspruch 2, wobei
eine Dicke des hinteren Endabschnitts (80) der Spitze (70) in Richtung des distalen Endes zunimmt.

## Revendications

1. Cathéter, comprenant :
une tige de cathéter (60) incluant
une couche interne (10) ;
une première couche de renforcement (20/22) enroulée sur une périphérie externe de la couche interne (10) ;
une couche intermédiaire (30) qui recouvre la première couche de renforcement (20/22) ;
une seconde couche de renforcement (40/42) enroulée sur une périphérie externe de la couche intermédiaire (30) ; et
une couche externe (50) qui recouvre la seconde couche de renforcement (40/42) ; et
une pointe (70) jointe à une extrémité distale de la tige de cathéter, **caractérisé en ce que**
la pointe (70) inclut une partie d'extrémité arrière (80) s'étendant dans une direction axiale et jointe à la couche intermédiaire (30) et à la couche externe (50), entre la première couche de renforcement (20/22) et la seconde couche de renforcement (40/42).

2. Cathéter selon la revendication 1, dans lequel
la couche intermédiaire (30) inclut une surface périphérique externe inégale,
la couche externe (50) inclut une surface périphérique interne inégale, et
la partie d'extrémité arrière (80) de la pointe (70) est jointe à au moins l'une parmi la surface périphérique externe de la couche intermédiaire (30) et la surface périphérique interne de la couche externe (50).

3. Cathéter selon la revendication 1 ou la revendication 2, dans lequel
une épaisseur de la partie d'extrémité arrière (80) de la pointe (70) est augmentée vers une direction d'extrémité distale.
